# EUROPÄISCHE PATENTSCHRIFT

(11) **EP 2 416 837 B1**
(45) Veröffentlichungstag und Bekanntmachung des Hinweises auf die Patenterteilung: **25.07.2018**
(21) Anmeldenummer: 10719247.8
(22) Anmeldetag: 03.04.2010
(51) Int. Cl.: A61M 39/00, A61M 39/10

(54) **VERBINDUNGSSTÜCK FÜR DEN SONDENSCHLAUCH EINER ENTERALEN ERNÄHRUNGSSONDE UND ANORDNUNG AUS EINER ENTERALEN ERNÄHRUNGSSONDE UND EINEM ENTERALEN ÜBERLEITSYSTEM**
CONNECTION PIECE FOR THE TUBE OF AN ENTERAL FEEDING TUBE AND ASSEMBLY OF AN ENTERAL FEEDING TUBE AND AN ENTERAL TRANSFER SYSTEM
ÉLEMENT DE RACCORD POUR UN TUBE D'UNE SONDE ENTÉRALE D'ALIMENTATION ET ENSEMBLE COMPOSÉ D'UNE SONDE D'ALIMENTATION ENTÉRALE ET D'UN SYSTÈME DE TRANSFERT ENTÉRAL

(30) Priorität: 07.04.2009 DE 102009016373
(43) Veröffentlichungstag der Anmeldung: 15.02.2012
(73) Patentinhaber: Fresenius Kabi Deutschland GmbH, 61352 Bad Homburg (DE)
(72) Erfinder: PECH, Bernhard, 65510 Hünstetten (DE); SCHÄFER, Helmut, 61250 Usingen (DE)
(74) Vertreter: Kusche, Robert
(86) Internationale Anmeldenummer: PCT/EP2010/002143
(87) Internationale Veröffentlichungsnummer: WO 2010/115598

(56) Entgegenhaltungen:
- EP-A1- 2 158 935
- WO-A1-96/14894
- US-A- 5 611 576
- US-A1- 2005 033 269
- US-A1- 2005 245 899

## Beschreibung

Die Erfindung bezieht sich auf ein Verbindungsstück für den Sondenschlauch einer enteralen Ernährungssonde, das ein proximales Anschlussstück zum Anschluss des Sondenschlauchs einer enteralen Ernährungssonde und ein distales Anschlussstück zum Anschluss eines Verbindungsstücks eines enteralen Überleitsystems aufweist. Darüber hinaus betrifft die Erfindung eine enterale Ernährungssonde mit einem Sondenschlauch, an dem das Verbindungsstück angeschlossen ist. Des Weiteren betrifft die Erfindung eine Anordnung aus einer enteralen Ernährungssonde und einem enteralen Überleitsystem.

Bei der enteralen Ernährung wird eine flüssige Nährlösung mit einer enteralen Ernährungssonde dem Patienten appliziert. Die enterale Nährlösung wird in einem Behältnis bereitgestellt, das über ein sogenanntes Überleitsystem mit der enteralen Ernährungssonde verbunden wird. Das enterale Überleitsystem weist eine Schlauchleitung mit einem proximalen und distalen Verbindungsstück auf. Das proximale Verbindungsstück wird mit der Ernährungssonde und das distale Verbindungsstück mit dem Nährlösungsbehältnis verbunden, so dass die Nährlösung aus dem Behältnis über die Schlauchleitung des Überleitsystems in die Ernährungssonde fließt.

Das Nährlösungsbehältnis wird in der Regel mehrmals täglich ausgetauscht, während beim Überleitsystem ein Wechsel alle 24 Stunden empfohlen wird. Die Ernährungssonde kann bis zu mehreren Monaten verwendet werden.

Zur Verbindung des Verbindungsschlauchs des Überleitsystems mit dem Sondenschlauch der Ernährungssonde finden im Stand der Technik die bekannten Luer-Lock-Konnektoren Verwendung, so dass Überleitsystem und Ernährungssonde miteinander verbunden werden können, ohne dass die Gefahr besteht, dass sich während der Applikation der Nährlösung der Sondenschlauch der Ernährungssonde von dem Verbindungsschlauch des Überleitsystems unbeabsichtigt löst. Nachteilig ist jedoch, dass die bekannten Luer-Lock-Verbindungen in der enteralen Applikationstechnik insofern ein Risiko für den Patienten darstellen können, als die Luer-Lock-Verbindungen in der enteralen Applikationstechnik mit Luer-Lock-Verbindungen verwechselt werden können, die insbesondere in der intravenösen Applikation und in der Beatmungstechnik eingesetzt werden. Wenn beispielsweise eine enterale Ernährung über eine intravenös gelegte Luer-Lock-Verbindung versehentlich appliziert werden sollte, besteht ein erhebliches Risiko für den Patienten.

Ein Sicherheitsgewinn wird im Stand der Technik dadurch erreicht, dass das enterale Überleitsystem ein weibliches Luer-Lock-Verbindungsstück aufweist, während die enterale Ernährungssonde ein männliches Luer-Lock-Verbindungsstück aufweist. Da intravenöse Sonden aber über ein weibliches Luer-Lock-Verbindungsstück verfügen, ist ausgeschlossen, dass ein enterales Überleitsystem direkt mit der intravenösen Sonde verbunden werden kann. Aufgrund der Vielzahl von verfügbaren Luer-Lock-Adaptern, die von den verschiedenen Herstellern angeboten werden, sind aber Verwechselungen nicht gänzlich auszuschließen.

Neben den Überleitsystemen und Ernährungssonden mit Luer-Lock-Konnektoren sind Ernährungssonden bekannt, die über ein trichterförmiges Verbindungsstück verfügen. Dabei bieten die verschiedenen Hersteller Ernährungssonden mit Anschlusstrichtern unterschiedlichen Durchmessers an. Um eine Verbindung mit den Anschlusstrichtern der bekannten Ernährungssonden herstellen zu können, sind im Stand der Technik stufenförmig ausgebildete Adapter bekannt, die im allgemeinen über eine Luer-Lock-Verbindung mit dem Verbindungsschlauch des Überleitsystems lösbar verbunden werden.

Die bekannten Stufenadapter haben den Vorteil, dass eine Verbindung mit Anschlusstrichtern von Ernährungssonden unterschiedlichen Durchmessers möglich ist. Der Stufenadapter wird in den Anschlusstrichter gesteckt, wobei in Abhängigkeit von dem Durchmesser des Anschlusstrichters derjenige Abschnitt des Stufenadapters, dessen Durchmesser dem Durchmesser des Anschlusstrichters entspricht, mit dem Anschlusstrichter in Kontakt kommt. Dabei werden die beiden Teile durch die Reibungskraft der Mantelflächen von Stufenadapter und Anschlusstrichter fixiert. Nachteilig ist, dass die Haltekraft zwischen Stufenadapter und Anschlusstrichter nicht ausreichend sein kann. Insbesondere bei der Verwendung von fetthaltigen Flüssignahrungen besteht das Risiko, dass sich die Verbindung unbeabsichtigt löst. Auch Alterungseffekte, die auf die Materialeigenschaften des Anschlusstrichters und seine Geometrie Einfluss haben, können zu einer reduzierten Haltekraft führen.

Schraubverbindungen, die auch im Bereich der enteralen Ernährung Verwendung finden, sind aus der WO 2005/055919 A1 bekannt. Nachteilig ist, dass die bekannten Schraubverbindungen nicht universell einsetzbar sind, sondern an die jeweilige Ernährungssonde bzw. das Überleitsystem, die über entsprechende Verbindungen verfügen, gebunden sind.

Die WO 2008/049568 A1 beschreibt ein Verbindungsstück, das ein Anschlussstück zum Anschluss eines Verbindungsstücks des Sondenschlauchs einer enteralen Ernährungssonde und ein Anschlussstück zum Anschluss des Verbindungsschlauchs eines Überleitsystems aufweist. Das Anschlussstück für das Verbindungsstück des Sondenschlauchs der enteralen Ernährungssonde verfügt über ein Arretierungselement, das derart ausgebildet ist, dass einerseits eine lösbare formschlüssige Verbindung mit einem Verbindungsstück eines Sondenschlauchs einer enteralen Ernährungssonde herstellbar ist, das ein mit dem Arretierungselement des Anschlussstücks komplementäres Arretierungselement aufweist, und das andererseits eine kraftschlüssige, aber nicht formschlüssige lösbare Verbindung auch mit Verbindungsstücken der Sondenschläuche anderer Ernährungssonden herstellbar ist, die nicht über ein derartiges Arretierungselement verfügen. Das Verbindungsstück ist ein einstückiges Spritzgießteil, das in großen Stückzahlen herstellbar ist.

Der Erfindung liegt die Aufgabe zugrunde, ein universell einsetzbares und einfach zu handhabendes Verbindungsstück für eine Sonde zur enteralen Ernährung bereitzustellen, das eine sichere, aber lösbare Verbindung von Überleitsystem und Ernährungssonde ermöglicht. Eine weitere Aufgabe der Erfindung liegt darin, eine universell einsetzbare und einfach zu handhabende Sonde zur enteralen Ernährung zu schaffen, die mit einem Überleitsystem sicher verbunden werden kann, ohne dass die Gefahr des unbeabsichtigten Lösens der Verbindung besteht. Darüber hinaus ist eine Aufgabe der Erfindung, eine universell einsetzbare und einfach zu handhabende Anordnung bestehend aus einer Ernährungssonde und einem Überleitsystem bereitzustellen, wobei sich Ernährungssonde und Überleitsystem sicher miteinander verbinden lassen.

Die Lösung dieser Aufgaben erfolgt erfindungsgemäß mit den in den Patentansprüchen 1, 16 und 17 angegebenen Merkmalen. Vorteilhafte Ausführungsformen sind Gegenstand der Unteransprüche.

Das erfindungsgemäße Verbindungsstück für ein enterales Überleitsystem verfügt über ein proximales Anschlussstück zum Anschluss eines Verbindungsstücks eines Sondenschlauchs einer enteralen Ernährungssonde und ein distales Anschlussstück zum Anschluss des Verbindungsschlauchs eines Überleitsystems.

Das erfindungsgemäße Verbindungsstück besteht aus zwei fest miteinander verbundenen Teilstücken, von denen das erste Teilstück aus einem Material besteht, das eine größere Härte als das Material hat, aus dem das zweite Teilstück des Verbindungsstücks besteht. Der zweiteilige Aufbau des Verbindungsstücks aus den beiden Werkstoffen unterschiedlicher Härte hat den Vorteil, dass die Materialeigenschaften der beiden Teilstücke den unterschiedlichen Anforderungen an die jeweiligen Verbindungen angepasst werden können. Beispielsweise kann mit dem Teilstück aus dem härteren Material eine vorzugsweise formschlüssige Verbindung und mit dem Teilstück aus dem weicheren Material eine vorzugsweise kraftschlüssige Verbindung zwischen dem erfindungsgemäßen Verbindungsstück und dem Verbindungsstück des Verbindungsschlauchs eines Überleitsystems geschaffen werden, wobei die formschlüssige Verbindung unnachgiebig und die kraftschlüssige Verbindung nachgiebig sein sollte. Die beiden Teilstücke aus Materialien unterschiedlicher Härte können beispielsweise durch Verschweißen oder Verkleben fest miteinander verbunden werden. Auch kann das Verbindungsstück mit den beiden Teilstücken als Mehrkomponenten-Spritzgussteil in großen Stückzahlen kostengünstig hergestellt werden.

Von Vorteil ist, dass das erfindungsgemäße Verbindungsstück Verwechselungen mit bei nicht enteralen Zugängen eingesetzten Luer-Lock-Verbindungen wegen der Inkompatibilität der an der Verbindung beteiligten Verbindungsstücke ausschließt. Bei einer bevorzugten Ausführungsform des Verbindungsstücks ist zumindest ein Teil des ersten Teilstücks des Verbindungsstücks, das aus einem härteren Material als das zweite Teilstück des Verbindungsstücks besteht, als ein Verriegelungselement ausgebildet, mit dem eine lösbare formschlüssige Verbindung mit einem Verbindungsstück eines Überleitsystems herstellbar ist, das ein mit dem Verriegelungselement der Ernährungssonde komplementäres Verriegelungselement aufweist. Das härtere Material des ersten Teilstücks stellt dabei sicher, dass das Verriegelungselement eine ausreichende Festigkeit hat, um eine formschlüssige Verbindung zu gewährleisten, die eine hohe Sicherheit hat. Das härtere Material hat auch den Vorteil, dass das Verriegelungselement nur einem geringen Verschleiß durch Abrasion unterworfen ist. Das Verriegelungselement, mit dem das Verbindungsstück des Verbindungsschlauchs eines enteralen Überleitsystems an dem Verbindungsstück des Sondenschlauchs einer enteralen Ernährungssonde gesichert wird, ist als Schraubverbindungselement mit einem Gewinde
ausgebildet
so dass sich das Verbindungsstück des enteralen Überleitsystem einfach und sicher mit dem Verbindungsstück der enteralen Ernährungssonde verbinden lässt. Allein entscheidend ist, dass die Verbindung eine formschlüssige lösbare Verbindung ist.

Das erste Teilstück des Verbindungsstücks besteht vorzugsweise aus einem Material, das aus der Gruppe ausgewählt ist, die Hart-Polyurethan, Hart-PVC, ABS und Polycarbonat sowie Verbindungen dieser Materialien mit anderen Materialien, beispielsweise Weichmacher etc. umfasst. Das Material, aus dem das zweite Teilstück des Verbindungsstücks besteht, ist vorzugsweise ein Material, das aus der Gruppe ausgewählt ist, die Weich-Polyurethan, Weich-PVC und Silikon sowie Verbindungen mit anderen Materialien, beispielsweise Weichmacher etc. umfasst.

Das Material des zweiten Teilstücks hat vorzugsweise eine Shore-Härte, die zwischen Shore A 90 und Shore A 30 liegt, wobei die Bestimmung der Shore-Härte nach DIN 53505 und DIN 7868 erfolgt.

Bei einer weiteren bevorzugten Ausführungsform ist zumindest ein Teil des zweiten Teilstücks des Verbindungsstücks, das aus einem weicheren Material besteht, als ein hohlförmiges Anschlussstück ausgebildet, mit dem eine lösbare kraftschlüssige Verbindung mit einem Verbindungsstück des Verbindungsschlauchs eines enteralen Überleitsystems herstellbar ist. Dabei hat das weichere, elastischere Material den Vorteil, dass eine sichere kraftschlüssige Verbindung auch dann herstellbar ist, wenn eine nicht optimale Passgenauigkeit der jeweiligen Verbindungsstücke gegeben ist, da sich das weichere Material an die geometrischen Besonderheiten unterschiedlicher Verbindungsstücke besser anpassen kann.

Das hohlförmige Anschlussstück des Verbindungsstücks weist vorzugsweise einen sich nach innen verjüngenden Aufnahmekanal auf, in den das Verbindungsstück des Verbindungsschlauchs eines enteralen Überleitsystems passend einsteckbar ist.

Eine weitere bevorzugte Ausführungsform des Verbindungsstücks sieht vor, dass das erste oder zweite Teilstück des Verbindungsstücks einen Teil aufweist, der als ein konisches Aufnahmestück ausgebildet ist, in den das Verbindungsstück des Verbindungsschlauchs eines enteralen Überleitsystems passend einsteckbar ist. Das konische Aufnahmestück gewährleistet eine sichere Abdichtung, schafft aber keine formschlüssige Verbindung wie der als Verriegelungselement ausgebildet6a Verbindungsstücks.

Eine weitere bevorzugte Ausführungsform des Verbindungsstücks sieht vor, dass das erste oder zweite Teilstück des Verbindungsstücks einen Teil aufweist, der als ein zylindrisches Befestigungsstück ausgebildet ist, an dem der Sondenschlauch einer enteralen Ernährungssonde befestigt wird.

Das konische Aufnahmestück für das Verbindungsstück des Verbindungsschlauchs eines enteralen Überleitsystems und das zylindrische Befestigungsstück für den Sondenschlauch einer enteralen Ernährungssonde können grundsätzlich Bestandteil des ersten oder zweiten Teilstücks sein. Für den Fall, dass das konische Aufnahmestück Bestandteil des ersten Teilstücks des Verbindungsstücks ist, das aus einem härteren Material besteht, sind das erste und zweite Teilstück des Verbindungsstücks vorzugsweise als hohlförmige Körper ausgebildet, die an den Enden fest miteinander verbunden sind. Bei dieser Ausführungsform bestehen nicht nur das Verriegelungselement, sondern auch das konische Aufnahmestück und das zylindrische Befestigungsstück aus einem härteren Material. Bei der alternativen Ausführungsform, bei der das konische Aufnahmestück und das zylindrische Befestigungsstück Bestandteil des zweiten Teilstücks sind, das aus einem weicheren Material besteht, sind das erste und zweite Teilstück des Verbindungsstücks vorzugsweise als hohlförmige Körper ausgebildet, wobei das erste Teilstück in das zweite Teilstück eingesetzt ist. Bei dieser Ausführungsform besteht nur das Verriegelungselement aus dem härteren Material, während alle übrigen Teile des Verbindungsstücks aus dem weicheren Material bestehen.

Das Verbindungsstück weist vorzugsweise eine das distale Anschlussstück verschließende Verschlusskappe auf, die mit einer Lasche unverlierbar an dem zweiten Teilstück des Verbindungsstücks befestigt ist, wobei Verschlusskappe und Lasche aus dem gleichen Material wie das zweite Teilstück des Verbindungsstücks bestehen. Damit besteht die Verschlusskappe mit der Lasche aus einem weicheren Material, das über eine ausreichende Flexibilität verfügt.

Bei einer besonders bevorzugten Ausführungsform besteht die Verschlusskappe aus einem Deckelteil mit einer Öffnung zum Anschluss einer Spritze und einem Verschlussteil zum Verschließen der Öffnung im Deckelteil, das mit einer Lasche unverlierbar an dem Deckelteil befestigt ist. Damit ist es möglich, nach dem Öffnen der auf das distale Anschlussstück aufgesetzten Verschlusskappe eine Flüssigkeit, insbesondere ein Medikament zuzuspritzen, wobei die Zuspritzöffnung wieder dicht verschlossen werden kann. Zum Zuspritzen einer Flüssigkeit, insbesondere eines Medikaments, ist bei einer alternativen Ausführungsform das Verbindungsstück mit einem zusätzlichen Anschlusstrichter vorgesehen, der mit einer Verschlusskappe verschließbar ist.

Die erfindungsgemäße Sonde zur enteralen Ernährung weist das erfindungsgemäße Verbindungsstück auf, das an den Sondenschlauch angeschlossen ist, während die erfindungsgemäße Anordnung die enterale Ernährungssonde, an deren Sondenschlauch das Verbindungsstück angeschlossen ist, und ein enterales Überleitsystem umfasst, an dessen Verbindungsschlauch ein Verbindungsstück angeschlossen ist, das mit dem Verbindungsstück der enteralen Ernährungssonde verbindbar ist.

Im Folgenden werden verschiedene Ausführungsbeispiele der Erfindung unter Bezugnahme auf die Zeichnungen näher erläutert.

Es zeigen:
- Fig. 1: eine erfindungsgemäße Anordnung bestehend aus einer enteralen Ernährungssonde und einem enteralen Überleitsystem,
- Fig. 2: das Verbindungsstück der enteralen Ernährungssonde in geschnittener perspektivischer Darstellung,
- Fig. 3: einen Schnitt durch die miteinander verbundenen Verbindungsstücke der Ernährungssonde und des Überleitsystems,
- Fig. 4: das Verbindungsstück des enteralen Überleitsystems in teilweises geschnittener perspektivischer Darstellung,
- Fig. 5: eine alternative Ausführungsform des erfindungsgemäßen Verbindungsstücks der enteralen Ernährungssonde in teilweise geschnittener perspektivischer Darstellung,
- Fig. 6: eine alternative Ausführungsform des Verbindungsstücks des enteralen Überleitsystems in teilweise geschnittener perspektivischer Darstellung und
- Fig. 7: eine weitere alternative Ausführungsform des erfindungsgemäßen Verbindungsstücks der enteralen Ernährungssonde in teilweise geschnittener perspektivischer Darstellung.

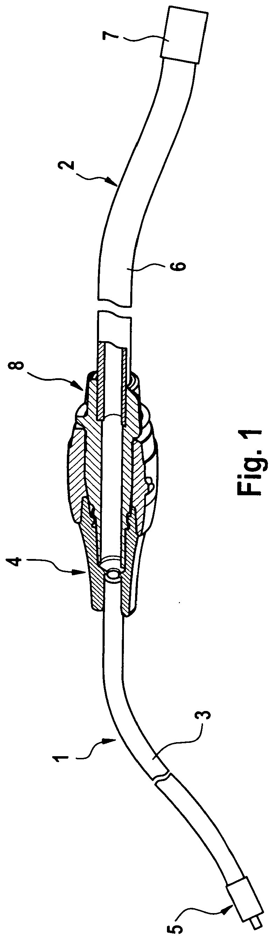

Fig. 1 zeigt eine erfindungsgemäße Anordnung bestehend aus einer enteralen Ernährungssonde 1 und einem enteralen Überleitsystem 2, die miteinander verbunden werden, um eine enterale Nährlösung aus einem nicht dargestellten Nährlösungsbehältnis einem Patienten zu applizieren. Da enterale Überleitsysteme und Ernährungssonden als solche dem Fachmann bekannt sind, beschränkt sich die Beschreibung im Wesentlichen auf die für die Erfindung relevanten Teile.

Die Ernährungssonde 1 weist einen Sondenschlauch 3 mit einem distalen Verbindungsstück 4 am distalen Schlauchende und einem in den Patienten einzuführenden proximalen Endstück 5 am proximalen Ende auf.

Das Überleitsystem 2 weist einen Verbindungsschlauch 6 mit einem distalen Verbindungsstück 7 am distalen Schlauchende und einem proximalen Verbindungsstück 8 am proximalen Schlauchende auf. Das distale Verbindungsstück 7 dient dem Anschluss des Überleitsystems an einen nicht dargestellten Nährlösungsbeutel, während das proximale Verbindungsstück 8 dem Anschluss der Ernährungssonde 1 dient. Hierfür wird das distale Verbindungsstück 4 der Ernährungssonde 1 mit dem proximalen Verbindungsstück 8 des Überleitsystems 2 verbunden.

Im Folgenden werden unterschiedliche Ausführungsbeispiele der Verbindungsstücke von Ernährungssonde 1 und Überleitsystem 2 im Einzelnen beschrieben.

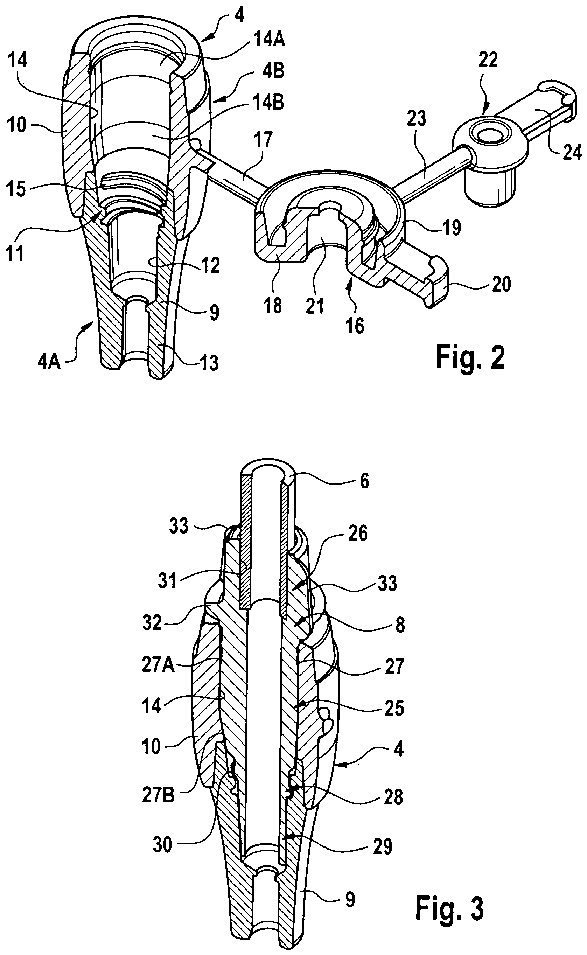

Fig. 2 zeigt ein erstes Ausführungsbeispiel des erfindungsgemäßen Verbindungsstücks 4, an dem das distale Ende des Sondenschlauchs 3 der Ernährungssonde 1 angeschlossen wird. Das Verbindungsstück 4 der Ernährungssonde 1 weist ein proximales Anschlussstück 4A für den Sondenschlauch der Ernährungssonde und ein distales Anschlussstück 4B für das Verbindungsstück des Verbindungsschlauchs des Überleitsystems auf. Das Verbindungsstück 4 besteht aus einem ersten Teilstück 9 und einem zweiten Teilstück 10, wobei die beiden Teilstücke 9, 10 fest miteinander verbunden sind. Das erste Teilstück 9 des Verbindungsstücks 4 besteht aus einem Material, das eine größere Härte als das Material hat, aus dem das zweite Teilstück 10 besteht. Das erste Teilstück 9 besteht beispielsweise aus Hart-Polyurethan, Hart-PVC, ABS (AcrylnitrilButadien-Styrol-Copolymerisat) oder Polycarbonat oder Verbindungen dieser Materialien mit weiteren Materialien, beispielsweise Weichmachern. Das zweite Teilstück 10 des Verbindungsstücks 4 besteht beispielsweise aus Weich-Polyurethan, Weich-PVC oder Silikon oder Verbindungen dieser Materialien mit anderen Materialien, beispielsweise Weichmachern. Das zweite Teilstück 10 des Verbindungsstücks hat eine Shore-Härte, die zwischen Shore A 90 und Shore A 30 liegt, wobei die Bestimmung der Shore-Härte nach DIN 53505 und DIN 7868 erfolgt.

Bei dem Ausführungsbeispiel von Fig. 1 ist ein Teil des ersten Teilstücks 9, das aus einem härteren Material besteht, als das zweite Teilstück 10, als ein Verriegelungselement 11 ausgebildet. Mit dem Verriegelungselement 11 kann eine lösbare formschlüssige Verbindung mit dem proximalen Verbindungsstück 8 des Überleitsystems 2 hergestellt werden, das ein mit dem Verriegelungselement 11 des Verbindungsstücks 4 der Ernährungssonde 1 komplementäres Verriegelungselement aufweist. Das proximale Verbindungsstück 8 des Überleitsystems 1 wird später noch im Einzelnen beschrieben.

Neben dem Verriegelungselement 11 weist das erste Teilstück 9 des Verbindungsstücks 4 ein konisches Aufnahmestück 12 auf, das einen Abschnitt des Verbindungsstücks 8 des Überleitsystems 2 passend aufnimmt. Das proximale Anschlussstück 4A des Verbindungsstücks 8 ist ein zylindrisches Befestigungsstück 13, in das der Sondenschlauch 3 der Ernährungssonde 1 eingeschoben wird. Der Sondenschlauch 3 der Ernährungssonde 1 kann mit dem zylindrischen Befestigungsstück 13 des Verbindungsstücks 4 verklebt oder verschweißt werden. Das Befestigungsstück 13 ist Bestandteil des ersten Teilstücks 9 des Verbindungsstücks 4.

Bei dem Ausführungsbeispiel von Fig. 1, bei dem nur ein Teil des ersten Teilstücks 9 als Verriegelungselement 11 ausgebildet ist, sind das erste und zweite Teilstück 9 und 10 des Verbindungsstücks 4 hohlförmige Körper aus unterschiedlichen Materialien, die an ihren Enden fest miteinander verbunden, beispielsweise verschweißt oder verklebt sind. Dabei übergreift ein außen liegender Ansatz des zweiten Teilstücks 10 einen innen liegenden Ansatz des ersten Teilstücks 9. Das Verbindungsstück 4 kann auch als ein Mehrkomponenten-Spritzgussteil hergestellt werden.
Der hohlförmige Körper des ersten Teilstücks 9 des Verbindungsstücks 4 weist an dem in Fig. 1 gezeigten oberen Abschnitt als Verriegelungselement ein Schraubgewinde 15 auf, das ein Innengewinde ist. An das Innengewinde 15 schließt sich im mittleren Abschnitt des hohlförmigen Körpers das konische Aufnahmestück 12 für das Verbindungsstück 8 des Überleitsystems 2 an. Das konische Aufnahmestück 12 geht in das zylindrische Befestigungsstück 13 am unteren Abschnitt des hohlförmigen Körpers über.

Das zweite Teilstück 10 des Verbindungsstücks 4, das aus einem weicheren Material als das erste Teilstück 9 besteht, weist einen Aufnahmekanal 14 auf, in den das Verbindungsstück 8 des Überleitsystems 1 passend einsteckbar ist. Der Aufnahmekanal 14 des zweiten Teilstücks 10 verjüngt sich in Richtung des ersten Teilstücks 9 des Verbindungsstücks 4. Der Aufnahmekanal 14 weist aus Gründen einer verbesserten Passgenauigkeit und eines somit verbesserten Kraftschlusses einen außen liegenden konischen Abschnitt 14A auf, an den sich ein innen liegender konischer Abschnitt 14B anschließt. Damit erhält das erste Teilstück 10 des Verbindungsstücks 4 die Form eines trichterförmigen Anschlussstücks 15 für das Verbindungsstück 8 des Überleitsystems 2.

Das Verbindungsstück 4 weist eine Verschlusskappe 16 auf, mit der das distale Anschlussstück 4B dicht verschlossen werden kann. Die Verschlusskappe 16 ist unverlierbar mit einer Lasche 17 an dem zweiten Teilstück 10 des Verbindungsstücks 4 befestigt. Verschlusskappe 16 und Lasche 17 bestehen aus dem gleichen weicheren Material wie das zweite Teilstück 10 des Verbindungsstücks 4, so dass sowohl das distale Anschlussstück 4B als auch die Verschlusskappe 16 mit der Lasche 17 eine ausreichende Flexibilität haben. Da das Verriegelungselement 11 hingegen aus einem härteren Material besteht, kann eine dauerhafte, verschleißarme und feste Verbindung hergestellt werden.

Die Verschlusskappe 16 weist einen Deckelteil 18 und einen Randteil 19 auf, an den eine kurze Lasche 20 angeformt ist, mit dem die Verschlusskappe 16 leicht von dem distalen Anschlussteil 4B des Verbindungsstücks 4 abziehbar ist.

In dem Deckelteil 18 der Verschlusskappe 16 befindet sich eine zentrale Öffnung 21 zum Anschluss einer nicht dargestellten Spritze, mit der eine Flüssigkeit, insbesondere ein Medikament zugespritzt werden kann. Die Öffnung 21 der Verschlusskappe 16 ist vorzugsweise konisch ausgebildet, um die konische Spitze der Spritze aufnehmen zu können.

Zum Verschließen der Öffnung 21 in der Verschlusskappe 16 ist ein passender Verschlussteil 22 vorgesehen, das mit einer Lasche 23 unverlierbar an der Verschlusskappe 16 angeformt ist. Auch an den Verschlussteil 22 ist wieder eine kurze Lasche 24 angeformt, mit der sich der Verschlussteil 22 aus der Öffnung 21 der Verschlusskappe 16 leicht herausziehen lässt. Die Verschlusskappe 16 mit den beiden Laschen 23 und 24 besteht wieder aus dem gleichen weicheren Material wie das distale Anschlussstück 4B des Verbindungsstücks 4.

Fig. 3 zeigt das proximale Verbindungsstück 8 des enteralen Überleitsystems 2, das mit dem distalen Verbindungsstück 4 der enteralen Ernährungssonde 1 fest verschraubt ist. Das proximale Verbindungsstück 8 des Überleitsystems 1 ist als ein langgestreckter hohlförmiger Körper ausgebildet, der ein proximales Teilstück 25 und ein distales Teilstück 26 aufweist. Das proximale Teilstück 25 weist einen oberen Abschnitt 27 auf, an den sich ein mittlerer Abschnitt 28 anschließt, der in einen unteren Abschnitt 29 übergeht. Der obere Abschnitt 27 des proximalen Teilstücks 25 weist aus Gründen einer verbesserten Passgenauigkeit und eines somit verbesserten Kraftschlusses einen oberen zylindrischen Abschnitt 27A auf, an den sich ein nach unten verjüngender unterer konischer Abschnitt 27B anschließt. Der mittlere Abschnitt 28 ist als Verriegelungselement 30 ausgebildet, das mit dem Verriegelungselement 11 des distalen Verbindungsstücks 4 der Ernährungssonde 1 komplementär ist. Bei dem vorliegenden Ausführungsbeispiel ist das Verriegelungselement ein Außengewinde 30, das in das Innengewinde 15 des Verbindungsstücks 4 der Ernährungssonde einschraubbar ist. Der untere zylindrische Abschnitt 29 des proximalen Teilstücks 25 ist passend in das konische Aufnahmestück 12 des Verbindungsstücks 4 der Ernährungssonde 1 einsetzbar, so dass beide Teile gegeneinander abdichten. Dadurch ist sichergestellt, dass das Verbindungsstück 8 des Überleitsystems 2 mit dem Verbindungsstück 4 der Ernährungssonde 1 flüssigkeitsdicht verbunden werden kann.

Fig. 3 zeigt, wie das Verbindungsstück 8 des Überleitsystems 2 in dem Verbindungsstück 4 der Ernährungssonde 1 sitzt. Der obere Abschnitt 27A des proximalen Teilstücks 25 sitzt in dem distalen Aufnahmestück 4B des Verbindungsstücks 4, während der untere Abschnitt 28 des proximalen Teilstücks 25 in dem konischen Aufnahmestück 12 des Verbindungsstücks 4 sitzt. Dabei kann die Abdichtung nicht nur an der Dichtfläche zwischen dem unteren zylindrischen Abschnitt 29 des proximalen Teilstücks 25 und dem konischen Aufnahmestück 12, sondern auch an der Dichtfläche zwischen dem oberen Abschnitt 27A des proximalen Teilstücks 25 und der Wand des Aufnahmekanals 14 des Verbindungsstücks 8 erfolgen. Da das Verbindungsstück im Bereich dieser Dichtfläche aus einem weicheren, nachgiebigeren Material besteht, wird eine sichere Abdichtung gewährleistet. Grundsätzlich kann auch eine Abdichtung über die Schraubverbindung erfolgen.

Das distale Teilstück 26 des proximalen Verbindungsstücks 8 weist einen zylindrischen Kanal 31 auf, in den das proximale Ende des Verbindungsschlauchs 6 des Überleitsystems passend eingeschoben ist. Der Verbindungsschlauch 6 kann mit dem distalen Teilstück 26 verklebt oder verschweißt werden. Mit einem umlaufenden Ansatz 32 stützt sich das distale Teilstück 26 des Verbindungsstücks 8 des Überleitsystems 2 an dem oberen Rand des distalen Anschlussstücks 4B des Verbindungsstücks 4 der Ernährungssonde 1 ab, wenn beide Verbindungsstücke 4, 8 fest miteinander verschraubt sind. Um das Verbindungsstück 8 des Überleitsystems 2 mit den Fingern besser greifen zu können, sind an dem distalen Teilstück 26 zwei radial abstehende Ansätze 33 angeformt.

Es ist ersichtlich, dass mit den beiden Verbindungsstücken 4, 8 eine lösbare formschlüssige Verbindung hergestellt werden kann. Die besondere Ausbildung des Verbindungsstücks 4 der Ernährungssonde 1 erlaubt nicht nur die Herstellung einer Verbindung mit dem besonderen Verbindungsstück 8 des Überleitsystems 1, sondern auch mit anderen Verbindungsstücken unterschiedlicher Ausbildung, so dass das Verbindungsstück universell einsetzbar ist. Auch ist der Anschluss der bekannten Stufenadapter möglich. Dies wird dadurch erreicht, dass das Verbindungsstück 4 der Ernährungssonde 1 über das trichterförmige distale Anschlussstück 4B verfügt, mit dem sich eine zwar nicht formschlüssige, aber kraftschlüssige Verbindung mit anderen Verbindungsstücken oder den bekannten Stufenadaptern herstellen lässt. Da das Anschlussstück aus einem weichen Material besteht, das eine ausreichende Flexibilität hat, kann sich das Anschlussstück den unterschiedlichen Durchmessern der anderen Verbindungsstücke anpassen. Dabei wird davon ausgegangen, dass aufgrund der sich nach innen verjüngenden Ausbildung des Aufnahmekanals 14 des Anschlussstücks 4B eine Dichtfläche zumindest in einem Teilabschnitt des Aufnahmekanals geschaffen wird.

Der zylindrische untere Abschnitt 29 des proximalen Teilstücks 25 des Verbindungsstücks 8 des Überleitsystems 2 weist eine geringere Wandstärke als der obere und mittlere Abschnitt 27 und 28 auf. Die Wandstärke des unteren Abschnitts 29 ist derart bemessen, dass sich das Verbindungsstück an diesem Abschnitt verformen lässt. Wenn das Verbindungsstück 8 des Überleitsystems 1 mit einem entsprechenden Verbindungsstück 4 einer Ernährungssonde 1 verbunden wird, verformt sich der untere Abschnitt 29 des Verbindungsstücks 8 derart, dass sich dieser Abschnitt in dem Verbindungsstück der Ernährungssonde "festkrallt". Eine derartige Verformung tritt insbesondere dann auf, wenn das Verbindungsstück 8 des Überleitsystems 2 beim Einstecken in das Verbindungsstück 4 der Ernährungssonde 1 leicht gedreht wird.

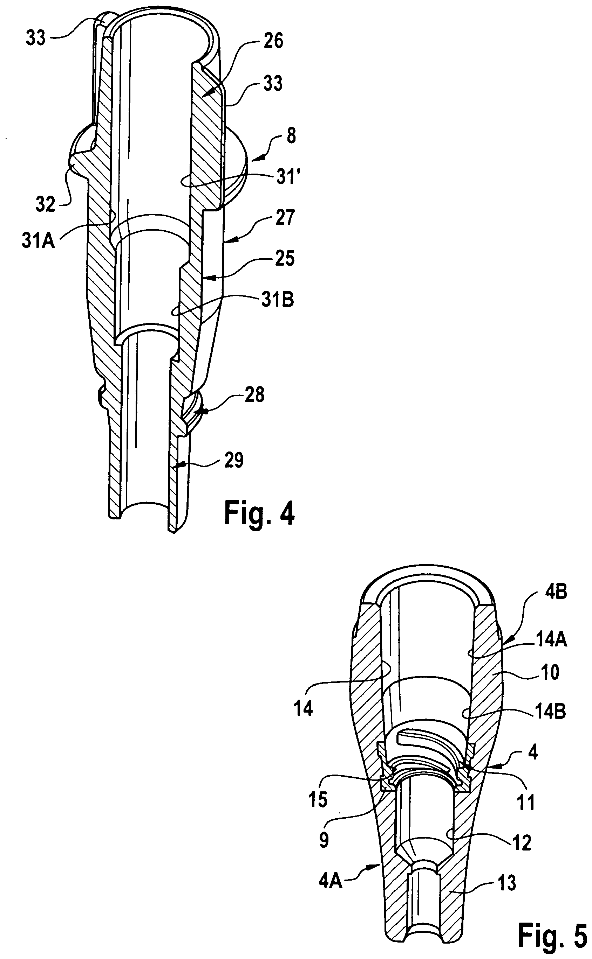

Fig. 4 zeigt eine alternative Ausführungsform des proximalen Verbindungsstücks 8 des enteralen Überleitsystems 2. Das Verbindungsstück unterscheidet sich von dem unter Bezugnahme auf Fig. 3 beschriebenen Verbindungsstück nur dadurch, dass sich der zylindrische Kanal 31' zur Aufnahme des Verbindungsschlauchs des Überleitsystems bis in den mittleren Abschnitt 28 des proximalen Teilstücks 25 des Verbindungsstücks 8 erstreckt. Darüber hinaus ist zylindrische Kanal 31 in zwei Abschnitte 31A und 31B unterschiedlichen Durchmessers unterteilt, wobei der Durchmesser sich zu dem mittleren Abschnitt 28 hin verringert. Die einander entsprechenden Teile sind daher mit den gleichen Bezugszeichen versehen.

Fig. 5 zeigt eine weitere Ausführungsform des distalen Verbindungsstücks 4 der enteralen Ernährungssonde 1. Das Ausführungsbeispiel von Fig. 5 unterscheidet sich von der unter Bezugnahme auf die Fig. 1 bis 3 beschriebenen Ausführungsform dadurch, dass nicht nur ein Teil des ersten Teilstücks 9, sondern das gesamte erste Teilstück als Verriegelungselement 11 ausgebildet ist, das aus einem härteren Material als das zweite Teilstück 10 des Verbindungsstücks 4 besteht. Die einander entsprechenden Teile sind wieder mit den gleichen Bezugszeichen versehen. Beide Teilstücke 9, 10 sind wieder hohlförmige Körper, wobei aber das erste Teilstück 9 in das zweite Teilstück 10 passend eingesetzt ist. Folglich besteht nur der Teil des Verbindungsstücks mit dem Innengewinde 15 aus dem härteren Material. Das konische Aufnahmestück 12 und das zylindrische Befestigungsstück 13 bestehen hingegen wie das distale Anschlussstück 4B aus dem weicheren Material.

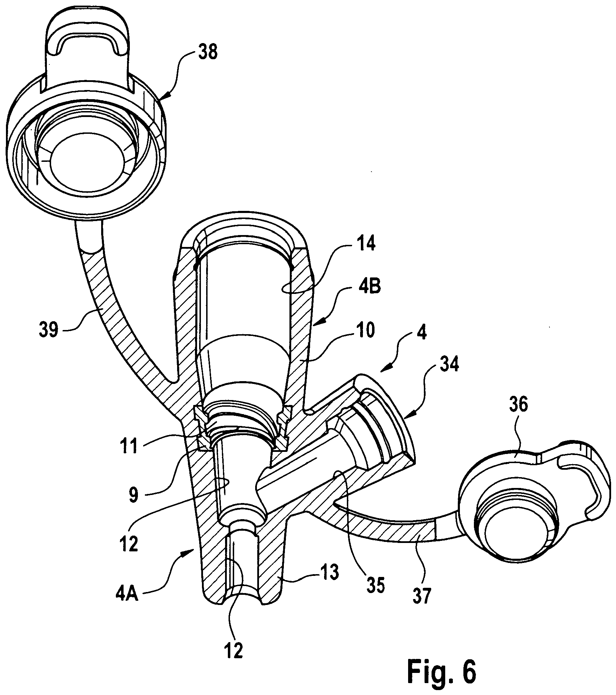

Fig. 6 zeigt eine weitere Ausführungsform des Verbindungsstücks 4 der Ernährungssonde 1. Die Ausführungsform von Fig. 6 unterscheidet sich von dem unter Bezugnahme auf Fig. 5 beschriebenen Ausführungsbeispiel dadurch, dass das Verbindungsstück 4 über einen zusätzlichen Anschlusstrichter 34 verfügt, der an das erste Teilstück 9 des Verbindungsstücks 4 angeformt ist. Bei dem Anschlusstrichter 34 handelt es sich um einen trichterförmigen Anschluss mit einer konischen Öffnung 35, in die sich die Spitze einer Spritze zum Zuspritzen eines Medikaments passend einstecken lässt. Der Anschlusstrichter 34 kann mit einer Verschlusskappe 36 verschlossen werden, die mit einer Lasche 37 mit dem Anschlusstrichter verbunden ist. Der Anschlusstrichter mit der Verschlusskappe und der Lasche bestehen wieder aus dem gleichen weicheren Material wie das zweite Teilstück 10 des Verbindungsstücks 4. Darüber hinaus verfügt die Ausführungsform von Fig. 6 über eine Verschlusskappe 38 zum Verschließen des distalen Anschlussstücks 15. Die Verschlusskappe 39 weist aber im Gegensatz zu der Verschlusskappe des unter Bezugnahme auf die Fig. 1 bis 3 beschriebenen Ausführungsbeispiels nicht eine Öffnung zum Zuspritzen einer Flüssigkeit auf. Die einander entsprechenden Teile sind wieder mit den gleichen Bezugszeichen versehen.

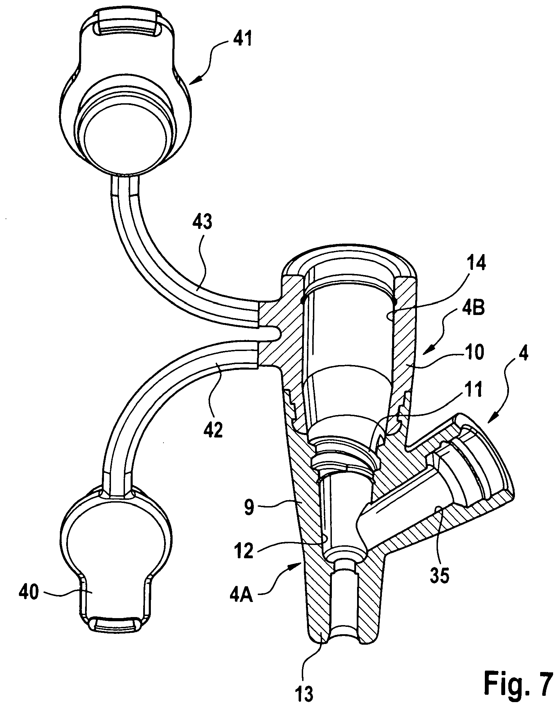

Fig. 7 zeigt eine weitere Ausführungsform des Verbindungsstücks 4 der Ernährungssonde 1. Die einander entsprechenden Teile sind wieder mit den gleichen Bezugszeichen versehen. Das Verbindungsstück von Fig. 7 unterscheidet sich von dem Verbindungsstück von Fig. 6 dadurch, dass sowohl das Verriegelungselement 11 als auch das konische Aufnahmestück 12 und das zylindrische Befestigungsstück 13 aus dem härteren Material bestehen, während das distale Anschlussstück 4B aus dem weicheren Material besteht. Insofern entspricht der Aufbau des Verbindungsstücks von Fig. 7 dem Verbindungsstück von den Fig. 1 bis 3. Die beiden Verschlusskappen 40 und 41 zum Verschließen des Anschlusstrichters 34 bzw. Anschlussstücks 4B sind unverlierbar mit Laschen 42, 43 an dem Verbindungsstück 4 befestigt, die an das distale Anschlussstück 4B des Verbindungsstücks angeformt sind.

## Patentansprüche

1. Verbindungsstück für den Sondenschlauch einer enteralen Ernährungssonde, das ein proximales Anschlussstück (4A) zum Anschluss des Sondenschlauchs einer enteralen Ernährungssonde und ein distales Anschlussstück (4B) zum Anschluss eines Verbindungsstücks eines enteralen Überleitsystems aufweist, wobei das Verbindungsstück (4) aus zwei fest miteinander verbundenen Teilstücken (9, 10) besteht, von denen das erste Teilstück (9) aus einem Material besteht, das eine größere Härte als das Material hat, aus dem das zweite Teilstück (10) des Verbindungsstücks besteht, wobei
zumindest ein Teil des ersten Teilstücks (9) des Verbindungsstücks (4) als ein Verriegelungselement (11) ausgebildet ist, mit dem eine lösbare formschlüssige Verbindung mit einem Verbindungsstück eines Überleitsystems herstellbar ist, das ein mit dem Verriegelungselement des Verbindungsstücks komplementäres Verriegelungselement aufweist,
**dadurch gekennzeichnet, dass** das Verriegelungselement (11) als Schraubverbindungselement, ausgebildet ist.

2. Verbindungsstück nach Anspruch 1, **dadurch gekennzeichnet, dass** das Material, aus dem das erste Teilstück (9) des Verbindungsstücks (4) besteht, ein Material ist, das aus der Gruppe ausgewählt ist, die Hart-Polyurethan, Hart-PVC, ABS und Polycarbonat umfasst.

3. Verbindungsstück nach Anspruch 1 oder 2, **dadurch gekennzeichnet, dass** das Material, aus dem das zweite Teilstück (10) des Verbindungsstücks (4) besteht, ein Material ist, das aus der Gruppe ausgewählt ist, die Weich-Polyurethan, Weich-PVC und Silikon umfasst.

4. Verbindungsstück nach einem der Ansprüche 1 bis 3, **dadurch gekennzeichnet, dass** das Material, aus dem das zweite Teilstück (10) des Verbindungsstücks (4) besteht, eine Shore-Härte hat, die zwischen Shore A 90 und Shore A 30 liegt.

5. Verbindungsstück nach einem der Ansprüche 1 bis 4, **dadurch gekennzeichnet, dass** zumindest ein Teil des zweiten Teilstücks (10) des Verbindungsstücks (4) als ein hohlförmiges Anschlussstück ausgebildet ist, mit dem eine lösbare kraftschlüssige Verbindung mit einem Verbindungsstück eines Überleitsystems herstellbar ist.

6. Verbindungsstück nach einem der Ansprüche 1 bis 5, **dadurch gekennzeichnet, dass** das hohlförmige Anschlussstück einen sich nach innen verjüngenden Aufnahmekanal (14) aufweist, in den ein Verbindungsstück eines Überleitsystems passend einsteckbar ist.

7. Verbindungsstück nach einem der Ansprüche 1 bis 6, **dadurch gekennzeichnet, dass** das erste Teilstück (9) des Verbindungsstücks (4) einen Teil aufweist, der als ein konisches Aufnahmestück (12) für das Verbindungsstück eines Überleitsystems ausgebildet ist.

8. Verbindungsstück nach einem der Ansprüche 1 bis 6, **dadurch gekennzeichnet, dass** das erste Teilstück (9) des Verbindungsstücks (4) einen Teil aufweist, der als zylindrisches Befestigungsstück (13) für einen Sondenschlauch einer enteralen Ernährungssonde ausgebildet ist.

9. Verbindungsstück nach Anspruch-7 oder 8, **dadurch gekennzeichnet, dass** das erste und zweite Teilstück (9, 10) des Verbindungsstücks (4) als hohlförmige Körper ausgebildet sind, die an den Enden fest miteinander verbunden sind.

10. Verbindungsstück nach einem der Ansprüche 1 bis 6, **dadurch gekennzeichnet, dass** das zweite Teilstück (10) des Verbindungsstücks 4) einen Teil aufweist, der als ein konisches Aufnahmestück (12) für das Verbindungsstück eines Überleitsystems ausgebildet ist.

11. Verbindungsstück nach einem der Ansprüche 1 bis 6, **dadurch gekennzeichnet, dass** das zweite Teilstück (10) des Verbindungsstücks (4) einen Teil aufweist, der als zylindrisches Befestigungsstück (13) für einen Sondenschlauch einer enteralen Ernährungssonde ausgebildet ist.

12. Verbindungsstück nach Anspruch 10 oder 11, **dadurch gekennzeichnet, dass** das erste und zweite Teilstück (9, 10) des Verbindungsstücks (4) als hohlförmige Körper ausgebildet sind, wobei das erste Teilstück (9) in das zweite Teilstück (10) eingesetzt ist.

13. Verbindungsstück nach einem der Ansprüche 1 bis 12, **dadurch gekennzeichnet, dass** das Verbindungsstück (4) ine Verschlusskappe (16) zum Verschließen des distalen Anschlussstücks (4B) aufweist, die mit einer Lasche (17) unverlierbar an dem zweiten Teilstück (10) des Verbindungsstücks befestigt ist, wobei Verschlusskappe (16) und Lasche (17) aus dem gleichen Material wie das zweite Teilstück (10) des Verbindungsstücks bestehen.

14. Verbindungsstück nach Anspruch 13, **dadurch gekennzeichnet, dass** die Verschlusskappe (16) aus einem Deckelteil (18) mit einer Öffnung (21) zum Anschluss einer Spritze und einem Verschlussteil (22) zum Verschließen der Öffnung im Deckelteil besteht, das mit einer Lasche (23) unverlierbar an dem Deckelteil befestigt ist.

15. Verbindungsstück nach einem der Ansprüche 1 bis 14, **dadurch gekennzeichnet, dass** das Verbindungsstück (4) einen mit einer Verschlusskappe (36) verschließbaren Anschlusstrichter (34) aufweist.

16. Enterale Ernährungssonde mit einem Sondenschlauch (3), der ein proximales und distales Ende aufweist, und einem Verbindungsstück (4) nach einem der Ansprüche 1 bis 15, wobei das distale Ende des Sondenschlauchs (3) der enteralen Ernährungssonde (1) an dem proximalen Anschlussstück (4A) des Verbindungsstücks (4) angeschlossen ist.

17. Anordnung aus einer enteralen Ernährungssonde nach Anspruch-16 und einem Überleitsystem (2), das einen Verbindungsschlauch (6) mit einem distalen und proximalen Ende aufweist, wobei an dem proximalen Ende des Verbindungsschlauchs ein Verbindungsstück (8) angeschlossen ist, das mit dem distalen Anschlussstück (4B) des Verbindungsstücks (4) der enteralen Ernährungssonde (1) verbindbar ist.

18. Anordnung nach Anspruch 17, **dadurch gekennzeichnet, dass** das Verbindungsstück (8) des Verbindungsschlauchs (6) des Überleitsystems (2) ein Verriegelungselement (30) aufweist, das mit dem Verriegelungselement (11) des Verbindungsstücks (4) des Sondenschlauchs (3) der enteralen Ernährungssonde (1) komplementär ausgebildet ist.

## Claims

1. Connection piece for the tube part of an enteral feeding tube, having a proximal attachment piece (4A) for the attachment of the tube part of an enteral feeding tube, and a distal attachment piece (4B) for the attachment of a connection piece of an enteral transfer system, wherein
the connection piece (4) is composed of two sections (9, 10) firmly connected to each other, of which the first section (9) is made of a material that is harder than the material from which the second section (10) of the connection piece is made, wherein at least part of the first section (9) of the connection piece (4) is designed as a locking element (11) with which it is possible to produce a releasable form-fit connection to a connection piece of a transfer system that has a locking element complementing the locking element of the connection piece, **characterized in that** the locking element (11) is designed as a screw connection element.

2. Connection piece according to Claim 1, **characterized in that** the material from which the first section (9) of the connection piece (4) is made is a material chosen from the group comprising rigid polyurethane, rigid PVC, ABS and polycarbonate.

3. Connection piece according to Claim 1 or 2, **characterized in that** the material from which the second section (10) of the connection piece (4) is made is a material chosen from the group comprising soft polyurethane, soft PVC and silicone.

4. Connection piece according to one of Claims 1 to 3, **characterized in that** the material from which the second section (10) of the connection piece (4) is made has a Shore hardness of between Shore A 90 and Shore A 30.

5. Connection piece according to one of Claims 1 to 4, **characterized in that** at least part of the second section (10) of the connection piece (4) is designed as a hollow attachment piece, with which it is possible to produce a releasable force-fit connection to a connection piece of a transfer system.

6. Connection piece according to one of Claims 1 to 5, **characterized in that** the hollow attachment piece has an inwardly narrowing receiving channel (14) into which a connection piece of a transfer system can be inserted with a snug fit.

7. Connection piece according to one of Claims 1 to 6, **characterized in that** the first section (9) of the connection piece (4) has a part designed as a conical receiving piece (12) for the connection piece of a transfer system.

8. Connection piece according to one of Claims 1 to 6, **characterized in that** the first section (9) of the connection piece (4) has a part designed as a cylindrical fastening piece (13) for a tube part of an enteral feeding tube.

9. Connection piece according to Claim 7 or 8, **characterized in that** the first and second sections (9, 10) of the connection piece (4) are designed as hollow bodies that are firmly connected to each other at the ends.

10. Connection piece according to one of Claims 1 to 6, **characterized in that** the second section (10) of the connection piece (4) has a part designed as a conical receiving piece (12) for the connection piece of a transfer system.

11. Connection piece according to one of Claims 1 to 6, **characterized in that** the second section (10) of the connection piece (4) has a part designed as a cylindrical fastening piece (13) for a tube part of an enteral feeding tube.

12. Connection piece according to Claim 10 or 11, **characterized in that** the first and second sections (9, 10) of the connection piece (4) are designed as hollow bodies, with the first section (9) being inserted into the second section (10).

13. Connection piece according to one of Claims 1 to 12, **characterized in that** the connection piece (4) has a closure cap (16) which is used to close the distal attachment piece (4B) and which, by way of a tab (17), is secured permanently to the second section (10) of the connection piece, said closure cap (16) and tab (17) being made of the same material as the second section (10) of the connection piece.

14. Connection piece according to Claim 13, **characterized in that** the closure cap (16) is composed of a lid part (18), with an opening (21) for the attachment of a syringe, and of a closure part (22) which is used to close the opening in the lid part and is secured permanently to the lid part by way of a tab (23).

15. Connection piece according to one of Claims 1 to 14, **characterized in that** the connection piece (4) has an attachment funnel (34) that can be closed by a closure cap (36).

16. Enteral feeding tube with a tube part (3), which has a proximal end and a distal end, and with a connection piece (4) according to one of Claims 1 to 15, the distal end of the tube part (3) of the enteral feeding tube (1) being attached to the proximal attachment piece (4A) of the connection piece (4).

17. Assembly composed of an enteral feeding tube according to Claim 16 and of a transfer system (2) that has a connection tube (6) with a distal end and a proximal end, the proximal end of the connection tube being connected to a connection piece (8) that can be connected to the distal attachment piece (4B) of the connection piece (4) of the enteral feeding tube (1).

18. Assembly according to Claim 17, **characterized in that** the connection piece (8) of the connection tube (6) of the transfer system (2) has a locking element (30), which is designed complementing the locking element (11) of the connection piece (4) of the tube part (3) of the enteral feeding tube (1).

## Revendications

1. Pièce de raccordement pour le tube de sonde d'une sonde entérale d'alimentation, qui présente une pièce de raccord proximale (4A) pour le raccord du tube de sonde d'une sonde entérale d'alimentation et une pièce de raccord distale (4B) pour le raccord d'une pièce de raccordement d'un système de transfert entéral, la pièce de raccordement (4) étant constituée de deux pièces partielles (9, 10) raccordées fixement l'une à l'autre, dont la première pièce partielle (9) se compose d'un matériau qui présente une plus grande dureté que le matériau constituant la deuxième pièce partielle (10) de la pièce de raccordement,
au moins une partie de la première pièce partielle (9) de la pièce de raccordement (4) étant réalisée sous forme d'élément de verrouillage (11) avec lequel peut être établie une liaison par engagement par correspondance de formes desserrable avec une pièce de raccordement d'un système de transfert qui présente un élément de verrouillage complémentaire à l'élément de verrouillage de la pièce de raccordement, **caractérisée en ce que** l'élément de verrouillage (11) est réalisé sous forme d'élément de raccordement par vissage.

2. Pièce de raccordement selon la revendication 1, **caractérisée en ce que** le matériau constituant la première pièce partielle (9) de la pièce de raccordement (4) est un matériau choisi parmi le groupe comprenant du polyuréthane dur, du PVC dur, de l'ABS et du polycarbonate.

3. Pièce de raccordement selon la revendication 1 ou 2, **caractérisée en ce que** le matériau constituant la deuxième pièce partielle (10) de la pièce de raccordement (4) est un matériau choisi parmi le groupe comprenant du polyuréthane mou, du PVC mou et de la silicone.

4. Pièce de raccordement selon l'une quelconque des revendications 1 à 3, **caractérisée en ce que** le matériau constituant la deuxième pièce partielle (10) de la pièce de raccordement (4) présente une dureté Shore qui est située entre Shore A 90 et Shore A 30.

5. Pièce de raccordement selon l'une quelconque des revendications 1 à 4, **caractérisée en ce qu'**au moins une partie de la deuxième pièce partielle (10) de la pièce de raccordement (4) est réalisée sous forme de pièce de raccord creuse avec laquelle peut être établie une liaison par engagement par force desserrable avec une pièce de raccordement d'un système de transfert.

6. Pièce de raccordement selon l'une quelconque des revendications 1 à 5, **caractérisée en ce que** la pièce de raccord creuse présente un canal de réception (14) se rétrécissant vers l'intérieur dans lequel peut être insérée avec ajustement une pièce de raccordement d'un système de transfert.

7. Pièce de raccordement selon l'une quelconque des revendications 1 à 6, **caractérisée en ce que** la première pièce partielle (9) de la pièce de raccordement (4) présente une partie qui est réalisée sous forme de pièce de réception conique (12) pour la pièce de raccordement d'un système de transfert.

8. Pièce de raccordement selon l'une quelconque des revendications 1 à 6, **caractérisée en ce que** la première pièce partielle (9) de la pièce de raccordement (4) présente une partie qui est réalisée sous forme de pièce de fixation cylindrique (13) pour un tube de sonde d'une sonde entérale d'alimentation.

9. Pièce de raccordement selon la revendication 7 ou 8, **caractérisée en ce que** la première et la deuxième pièce partielle (9, 10) de la pièce de raccordement (4) sont réalisées sous forme de corps creux qui sont raccordés fixement l'un à l'autre aux extrémités.

10. Pièce de raccordement selon l'une quelconque des revendications 1 à 6, **caractérisée en ce que** la deuxième pièce partielle (10) de la pièce de raccordement (4) présente une partie qui est réalisée sous forme de pièce de réception conique (12) pour la pièce de raccordement d'un système de transfert.

11. Pièce de raccordement selon l'une quelconque des revendications 1 à 6, **caractérisée en ce que** la deuxième pièce partielle (10) de la pièce de raccordement (4) présente une partie qui est réalisée sous forme de pièce de fixation cylindrique (13) pour un tube de sonde d'une sonde entérale d'alimentation.

12. Pièce de raccordement selon la revendication 10 ou 11, **caractérisée en ce que** la première et la deuxième pièce partielle (9, 10) de la pièce de raccordement (4) sont réalisées sous forme de corps creux, la première pièce partielle (9) étant insérée dans la deuxième pièce partielle (10).

13. Pièce de raccordement selon l'une quelconque des revendications 1 à 12, **caractérisée en ce que** la pièce de raccordement (4) présente un capuchon de fermeture (16) pour fermer la pièce de raccord distale (4B), qui est fixé avec une patte (17) de manière imperdable à la deuxième pièce partielle (10) de la pièce de raccordement, le capuchon de fermeture (16) et la patte (17) se composant du même matériau que la deuxième pièce partielle (10) de la pièce de raccordement.

14. Pièce de raccordement selon la revendication 13, **caractérisée en ce que** le capuchon de fermeture (16) se compose d'une partie de couvercle (18) avec une ouverture (21) pour le raccord d'une seringue et une partie de fermeture (22) pour fermer l'ouverture dans la partie de couvercle, qui est fixée avec une patte (23) de manière imperdable à la partie de couvercle.

15. Pièce de raccordement selon l'une quelconque des revendications 1 à 14, **caractérisée en ce que** la pièce de raccordement (4) présente un entonnoir de raccordement (34) pouvant être fermé avec un capuchon de fermeture (36).

16. Sonde entérale d'alimentation comprenant un tube de sonde (3) qui présente une extrémité proximale et une extrémité distale, et une pièce de raccordement (4) selon l'une quelconque des revendications 1 à 15, l'extrémité distale du tube de sonde (3) de la sonde entérale d'alimentation (1) étant raccordée à la pièce de raccord proximale (4A) de la pièce de raccordement (4).

17. Agencement constitué d'une sonde entérale d'alimentation selon la revendication 16 et d'un système de transfert (2) qui présente un tube de raccordement (6) ayant une extrémité proximale et une extrémité distale, une pièce de raccordement (8) étant raccordée à l'extrémité proximale du tube de raccordement, laquelle peut être raccordée à la pièce de raccord distale (4B) de la pièce de raccordement (4) de la sonde entérale d'alimentation (1).

18. Agencement selon la revendication 17, **caractérisé en ce que** la pièce de raccordement (8) du tube de raccordement (6) du système de transfert (2) présente un élément de verrouillage (30) qui est réalisé de manière complémentaire avec l'élément de verrouillage (11) de la pièce de raccordement (4) du tube de sonde (3) de la sonde entérale d'alimentation (1).
